# EUROPÄISCHE PATENTSCHRIFT

(11) **EP 0 419 613 B1**
(45) Veröffentlichungstag und Bekanntmachung des Hinweises auf die Patenterteilung: **23.11.1994**
(21) Anmeldenummer: 90904782.1
(22) Anmeldetag: 30.03.1990
(51) Int. Cl.: A61L 2/18, G02C 13/00

(54) **GERÄT ZUR PFLEGE VON KONTAKTLINSEN**
DEVICE FOR CLEANING CONTACT LENSES
APPAREIL D'ENTRETIEN POUR LENTILLES DE CONTACT

(30) Priorität: 05.04.1989 CH 1261/89
(43) Veröffentlichungstag der Anmeldung: 03.04.1991
(73) Patentinhaber: Zimmerli, Edwin, CH-8634 Hombrechtikon (CH)
(72) Erfinder: Zimmerli, Edwin, CH-8634 Hombrechtikon (CH)
(74) Vertreter: Felber, Josef
(86) Internationale Anmeldenummer: CH9000083
(87) Internationale Veröffentlichungsnummer: WO9011785

(56) Entgegenhaltungen:
- EP-A- 0 218 539
- US-A- 4 381 285

## Beschreibung

Die vorliegende Erfindung betrifft ein Gerät, welches die Kontaktlinsenpflege mittels zweier verschiedener Reinigungsflüssigkeiten erleichtert. Wenn zwei solche verschiedene Flüssigkeiten zur Pflege von Kontaktlinsen verwendet werden, spricht man von zweistufigen Pflegesystemen. Diese arbeiten grundsätzlich mit Wasserstoffperoxid. Im ersten Schritt, beziehungsweise in der ersten Stufe, erfolgt die Desinfektion und gleichzeitig eine gewisse Reinigung der Kontaktlinsen mit Wasserstoffperoxid. Nach einer Umlagerung der Kontaktlinsen in ein Gefäss mit der zweiten Flüssigkeit oder nach einem Lösungswechsel im selben Behältnis erfolgt im zweiten Schritt die Neutralisation der Kontaktlinsen von unverbrauchten Wasserstoffperoxid-Resten. Solche zweistufig arbeitende Wasserstoffperoxid-Pflegesysteme bestehen deshalb üblicherweise aus drei Elementen. Erstens wird ein Kunststoffbehälter mit einer in dessen abnehmbarem Deckel angebrachten Halterung zur Aufnahme der Kontaktlinsen abgegeben. Zweitens wird für die erste Stufe eine Flasche mit der Reinigungsflüssigkeit (Wassersotffperoxidlösung) für die Desinfektion und Reinigung der Kontaktlinsen abgegeben. Drittens wird für die zweite Stufe eine Flasche mit Neuralisationsflüssigkeit für die Neutralisation und Aufbewahrung der Kontaktlinsen abgegeben. Bei der Neutralisations-Flüssigkeit handelt es sich um eine auf den pH-Wert der Tränenflüssigkeit der Augen abgestimmte physiologische Kochsalzlösung. Zur Vornahme der zweistufigen Reinigung wird der Kunststoffbehälter mit der im Deckel integrierten Halterung für die Aufnahme der Kontaktlinsen verwendet. Es wird nun folgendermassen vorgegangen: Die Kontaktlinsen werden in die im Deckel des Kunststoffbehälters integrierte Halterung eingelegt. Der Behälter wird mit Reinigungsflüssigkeit gefüllt und dann mit dem Deckel verschlossen. Nun müssen die Kontaktlinsen für die vorgeschriebene Zeit von zum Beispiel 20 Minuten in der Reinigungsflüssigkeit verbleiben. Danach wird die Reinigungsflüssigkeit weggeschüttet. Der Behälter wird jetzt mit Neutralisationsflüssigkeit gefüllt und wieder mit dem Deckel verschlossen. Die Kontaktlinsen müssen während einer vorgeschriebenen Mindestzeit neutralisiert werden. Bei dieser Pflege der Kontaktlinsen müssen die Anweisungen der Pflegemittelhersteller peinlich genau befolgt werden, um Entzündungen der Augen und Beschädigungen der Kontaktlinsen mit Sicherheit zu vermeiden. Viele Kontaktlinsenarten müssen täglich gepflegt werden. Die Pflege erfolgt aus praktischen Gründen in den meisten Fällen vor dem Schlafengehen, wenn der Kontaktlinsenträger nicht mehr auf die Linsen angewiesen ist. Es kommt vor, dass er während der vorgeschriebenen Reinigungszeit bereits einschläft, das Wechseln der Kontaktlinsen von der einen Lösung in die andere vergisst oder die aufwendigen Pflegevorschriften nicht genau befolgt. Wird die Neutralisation vergessen oder wird die vorgeschriebene Neutralisationszeit nicht eingehalten, so kann die verbleibende Restaktivität des Wasserstoffperoxids an den Kontaktlinsen bei deren Tragen unangenehme Folgen für die Augen nach sich ziehen. Diese Folgen reichen von leichten Augenreizungen bis hin zu ernsthaften Schädigungen des Hornhautepithels.

Aus der EP-A-0'218'539 ist ein Reinigungsgerät bekannt, welches einen Schwenkarm aufweist, der Halterungen für die Kontaktlinsen trägt. Dieser Schwenkarm kann mittels eines elektro-mechanischen Antriebes über ein Zahnstangengetriebe so bewegt werden, dass die Halterungen mit den von ihnen getragenen Kontaktlinsen nach Verweilen während einer einstellbaren Zeitdauer in einem ersten Behälter aus diesem herausgehoben und in einen zweiten Behälter geschwenkt werden. Die Nachteile dieses Gerätes bestehen darin, dass der Antriebsmechanismus nicht lautlos arbeitet, recht aufwendig und teuer in der Herstellung ist, und ausserdem der Bedienungskomfort des Gerätes noch zu wünschen übrig lässt.

Aus der US-A-4'381'285 ist ein wieteres Reinigungsgerät bekannt, bei dem die Kontaktlinsen in kleine, perforierte Aufnahmebehälter gelegt werden, welche dann auf einen Wurfarm gelegt werden. In der Ausgangsposition befindet sich der Wurfarm mit den daraufliegenden Aufnahmebehältern eingetaucht in der ersten Reinigungsflüssigkeit. Nach einer einstellbaren Zeit wird der Wurfarm mittels eines Federmechanismus um eine horizontal liegende Achse hochgeschleudert und wirft die Aufnahmebehälter mitsamt den darin enthaltenen Kontaktlinsen in die zweite Reinigungsflüssigkeit. Dieses Gerät hat die Nachteile, dass das Wechseln der Kontaktlinsen ein störendes Schnappgeräusch entwickelt und dass die Kontaktlinsen dabei mechanische Schläge erleiden. Ausserdem müssen die Aufnahmebehälter mit den gereinigten Kontaktlinsen von Hand oder mit einem Instrument aus der zweiten Flüssigkeit herausgefischt werden.

Es ist daher die Aufgabe der vorliegenden Erfindung, ein Gerät zu schaffen, welches bei der Benützung eines zweistufigen Kontaktlinsen-Pflegesystems die Desinfektion, Reinigung und Neutralisation zuverlässig und selbständig durchführt, ohne dass der Kontaktlinsenträger das Wechseln der Linsen von der Reinigungslösung in die Neutralisationslösung durchführen oder überwachen muss.

Diese Aufgabe löst ein Gerät zur Pflege von Kontaktlinsen mittels zwei verschiedenen Flüssigkeiten, bestehend aus einem Gehäuse mit zwei Behältnissen zur Aufnahme der zwei verschiedenen Flüssigkeiten und einer darauf beweglich angeordneten Halterung zum abwechslungsweisen und selbsttätigen Eintauchen der von ihr gehaltenen Kontaktlinsen in die beiden Behältnisse, wobei diese Halterung auf einer Achse bezüglich des Gehäuses schwenkbar gelagert ist, und das sich dadurch auszeichnet, dass eine Drehplatte vorhanden Ist, welche die Halterung trägt, und dass die Drehplatte auf einer Führungsstange gehalten ist, die drehbar und verschiebbar in einer Bohrung im Gehäuse sitzt, welche zwischen den beiden Behältnissen angeordnet ist.

Vorteilhafte Ausgestaltungen der Erfindung gehen aus den ab-hängigen Ansprüchen hervor und sind in der nachfolgenden Beschreibung erläutert. In der Zeichnung ist ein Ausführungsbeispiel dargestellt, das in der Beschreibung in seinem Aufbau und seiner Funktion erklärt wird.

Es zeigt:
- Figur 1: eine perspektivische Gesamtansicht des Reinigungsgerätes mit abgehobener Drehplatte;
- Figur 2: eine schematische Darstellung des mechanischen Betätigungsmechanismus mit Kraftspeicher und elektrischem Auslösemechanismus nach dem Inbetriebsetzen mit den Linsen in der Reinigungsflüssigkeit;
- Figur 3: eine schematische Darstellung des mechanischen Betätigungsmechanismus mit Kraftspeicher und elektrischen Auslösemechanismus beim Wechseln vor der Drehung der Drehplatte;
- Figur 4: eine schematische Darstellung des mechanischen Betätigungsmechanismus mit Kraftspeicher und elektrischen Auslösemechanismus in der Endlage mit den Linsen in der Neutralisationsflüssigkeit.

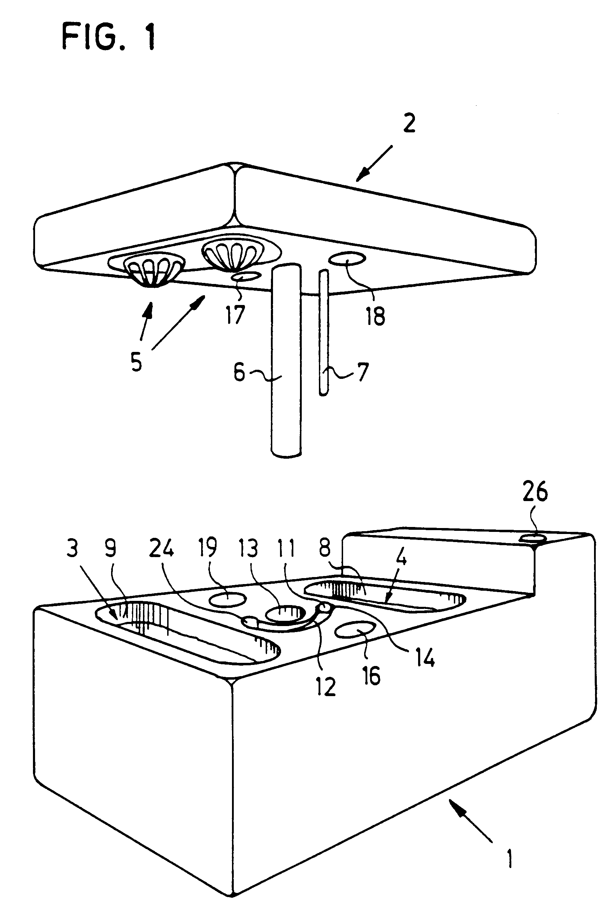

Das erfindungsgemässe Gerät gemäss Figur 1 schliesst ein Gehäuse 1 ein, welches ein Behältnis 9 für die Reinigungsflüssigkeit 3 und ein Behältnis 8 für die Neutralisationsflüssigkeit 4 aufweist, sowie eine Drehplatte 2, an deren Unterseite sich die Halterung 5 zur Aufnahme der Kontaktlinsen befindet.

Diese Halterung 5 besteht vorteilhaft aus zwei korbähnlichen Behältern, die entweder zum Einlegen der Kontaktlinsen zum Beispiel leicht von der Drehplatte 2 wegnehmbar und wieder an sie anbringbar sind, oder an der Drehplatte 2 abschwenkbar befestigt sind. In der abgeschwenkten Lage können dann die Kontaktlinsen eingelegt werden, wonach die Behälter wiederum aufgeschwenkt werden und in dieser Lage mittels eines Schnappverschlusses in geschlossener Lage an der Drehplatte gehalten sind. Die Drehplatte 2 ist auf einer Führungsstange 6 befestigt, welche in einer Bohrung 13 im Gehäuse 1 verschiebbar und drehbar gehalten ist. Die beiden Behältnisse 8 und 9 sind symmetrisch zu dieser Bohrung 13 angeordnet und bilden hier mit dem Gehäuse 1 eine Baueinheit. Selbstverständlich können aber die eigentlichen Behältnisse zur Aufnahme der beiden Flüssigkeiten auch von gesonderten Behältern gebildet sein, die dann in die Behältnisse 8,9 im Gehäuse einlegbar sind. Dieses ist sogar vorteilhaft, weil solche Einlagebehältnisse leichter zu reinigen sind, indem man sie vom eigentlichen Gerät wegnehmen und zum Desinfizieren in kochendem Wasser aussieden kann. Solche Einlagebehältnisse bestehen vorteilhaft aus gummielastischem Material, zum Beispiel aus dem handelsüblichen Material Lupolen 1810H. Infolge ihrer gummielastischen Eigenschaft können diese Einlagebehältnisse an ihrem Rand einen Wulst aufweisen, der bei ihrer Einlage auf dem Rand der Gehäusebehältnisse 8,9 aufliegt. Wird die Drehplatte 2 ganz auf die Wulste der Einlagebehältnisse hinuntergedrückt und in dieser Position gesichert, so sind die Einlagebehältnisse dichtend verschlossen, sodass die von ihnen enthaltene Flüssigkeit auch beim Stürzen des Gerätes dicht eingeschlossen ist. Damit kann das Gerät auch problemlos auf Reisen mitgenommen werden. Zur Sicherung der Drehplatte 2 in ihrer untersten Position, bei der sie auf die Wulste der Einlagebehältnisse drückt, kann auf beiden Seiten des Gehäuses je ein nicht dargestellter Schiebeverschluss angeordnet sein, dessen leicht keilförmige Schieber beim Verriegeln eine die Drehplatte 2 auf das Gehäuse pressende Kraft bewirken. Für die Funktion des Gerätes sind in der Drehplatte 2 Permanentmagnete 17 und 18 eingelegt, die mit den Metallkernen 16 und 19 im Gehäuseteil zusammenwirken, wie das nachfolgend noch erläutert wird. Neben der Führungsstange 6 an der Drehplatte 2 ist parallel zu ihr eine Schubstange 7 angeordnet, welche je nach Schwenklage der Drehplatte 2 in bezug auf das Gehäuse 1 beim Hinunterdrücken der Drehplatte 2 entweder in die Bohrung 14 oder die Bohrung 24 einpasst. Die Bohrung 14 enthält eine Druckfeder als Kraftspeicher, auf der eine Kugel 11 sitzt. Vom oberen Randbereich der Bohrung 14 führt eine konzentrisch um die zentrale Bohrung 13 verlaufende Nut 12 mit schraubenlinienförmig abfallendem Nutboden in die Bohrung 24 hinein.

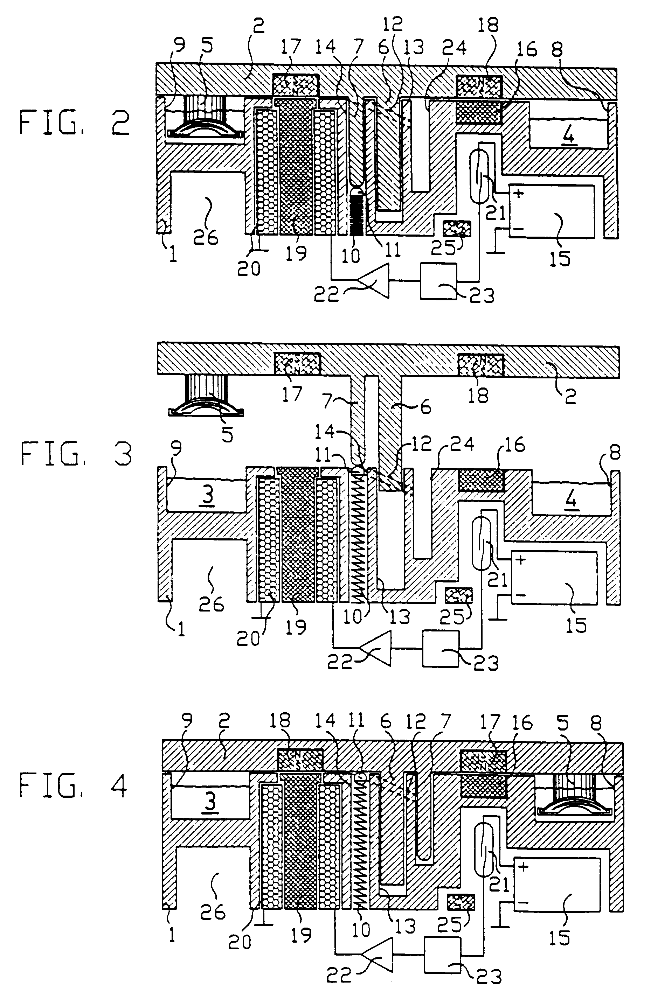

Anhand der schematischen Zeichnungen in den Figuren 2,3 und 4, welche je einen Querschnitt durch das Gerät darstellen, wird nachfolgend die Inbetriebsetzung und die Funktion des Gerätes erläutert, wobei die Bedeutung der einzelnen, oben beschriebenen Elemente klar wird. Das Behältnis 9 oder das in diesem befindliche Einlagebehältnis enthält eine Reinigungsflüssigkeit 3, das Behältnis 8, beziehungsweise das in ihm befindliche Einlagebehältnis, eine Neutralisationsflüssigkeit 4. Die Figur 4 zeigt das Gerät in der Endlage, in der die Kontaktlinsen, beziehungsweise die für sie vorgesehene Halterung 5, in die Neutralisationsflüssigkeit eingetaucht sind. Diese Endlage entspricht zugleich auch der Ausgangslage zur Inbetriebsetzung des Gerätes, die es bei Nichtgebrauch einnimmt. Für die Inbetriebsetzung des Gerätes wird, ausgehend aus dieser Lage gemäss Figur 4, die Drehplatte 2 aus der Bohrung 13 herausgezogen und die zu reinigenden Kontaktlinsen werden in die Halterung 5 eingelegt. Danach wird die mit der Drehplatte 2 verbundene Führungsstange 6 so in die Bohrung 13 eingeführt, wie das Figur 2 zeigt. Die Drehplatte 2 ist dabei in bezug auf das Gehäuse 1 in einer solchen Schwenklage, dass die Schubstange 7 beim Hinunterdrücken der Drehplatte 2 in die Bohrung 14 geschoben wird und den darin angeordneten Kraftspeicher des Betätigungsmechanismus spannt. Im vorliegenden Fall handelt es sich bei diesem Kraftspeicher um eine einfache Druckfeder 10, auf welcher zudem eine Kugel 11 angeordnet ist. Wie Figur 2 zeigt, ist die Halterung 5, in der die Kontaktlinsen liegen, in die Reinigungsflüssigkeit eingetaucht, welche sich im Behältnis 9 befindet. In der Tieflage der Drehplatte 2 nach Figur 2 wird gleichzeitig ein zeitabhängig gesteuerter Auslösemechanismus in Betrieb gesetzt. Dieser elektrische Auslösemechanismus ist in Figur 2 bis Figur 4 schematisch dargestellt und funktioniert wie folgt: In der Drehplatte 2 sind Permanentmagnete 17,18 mit zueinander entgegengesetzter Polung eingelegt, wie das anhand der schematischen Darstellungen nach Figur 2,3 und 4 ersichtlich ist.

In der eben eingenommenen Lage nach Figur 2, in der die Kraftspeicherfeder 10 des Betätigungsmechanismus gespannt ist, ziehen sich die Permanentmagnete 17,18 an die Metallkerne 16,19 an und vermögen die Drehplatte 2 gegen die Kraft der Feder 10 in ihrer unteren Lage zu halten. Gleichzeitig wird durch die Wirkung des Permanentmagneten 18 ein berührungsloser Reed-Schalter 21 geschlossen, sodass der Stromkreis über eine eingebaute Batterie 15 geschlossen wird. Dadurch wird ein Zeitelement 23 gestartet, das ein entsprechend beschalteter integrierter Schaltkreis sein kann. An diesem können beliebige Zeitabschnitte eingestellt werden, nach deren Ablauf der Treiber 22 gespeist wird. Nach Ablauf der eingestellten Zeit wird deshalb über den Treiber 22 die Spule 20 mit dem Kern 19 erregt, der oben bündig mit der Gehäuseoberseite endet. Durch die Erregung der Spule 20 wird ein Magnetfeld aufgebaut, das dem Magnetfeld des Permanentmagneten 17 entgegenwirkt. Der Permanentmagnet 18 alleine vermag nun die Kraft der gespannten Feder 10 nicht mehr zu kompensieren, so dass der Betätigungsmechanismus freigegeben wird. Die Feder 10 mit der darauf sitzenden Kugel 11 drückt in der Folge die Schubstange 7 und damit die Drehplatte 2 nach oben. Dabei aber wird die Halterung 5 aus dem Reinigungsbehältnis 9 gehoben. Am Ende der Bohrung 14, in welcher die Feder 10 und die Kugel 11 untergebracht sind, rutscht die unten angeschrägte oder abgerundete Schubstange 7 über die Kugel 11 in die schraubenlinienförmige Nut 12 (siehe auch Figur 1) und gleitet infolge der Schwerkraft der ganzen Drehplatte 2 entlang der Nut 12 in die Bohrung 24. Die schraubenlinienförmige Nut 12 ist konzentrisch um die Bohrung 13 für die Führungsstange 6 angeordnet. Sie beginnt bei der Bohrung 14 und mündet nach einem Halbkreis von 180° in die Bohrung 24. Die ganze Drehplatte 2 wird daher kraft ihrer Schwerkraft um 180° gedreht, bis die Schubstange 7 in die Bohrung 24 sinkt und die Drehplatte 2 schliesslich die Position gemäss Figur 4 einnimmt. Damit gelangen die in der Halterung 5 untergebrachten Kontaktlinsen in das Neutralisationsbehältnis 8, wie das die Figur 4 zeigt. Im Neutralisationsbehältnis 8 oder dem entsprechenden Einlagebehältnis kann ein mit Platinschwamm oder Platinmohr beschichteter Kunststoffkörper als Katalysator eingelegt sein, welcher die Wasserstoffperoxidreste in Wasser- und Sauerstoff aufspaltet, bis eine schwache Kochsalzlösung übrigbleibt. Der während des Wechselvorganges entstehende Ueber- beziehungsweise Unterdruck in der Bohrung 13 verlangsamt und dämpft das Heben und Senken der Drehplatte 2. Das Schliessen des berührungslosen Reedschalters 21 in der Position von Figur 4 wird durch magnetisches Vorspannen desselben mittels des unter dem Reedschalter 21 angeordneten Permanentemagneten 25 verhindert. Der Reedschalter 21, der nahe unter dem Metallkern 16 parallel zur Spule 20 liegt, wird nur beim Schwenken und Herunterdrücken der Drehplatte 2 gegen die Kraft der Druckfeder 10 geschaltet, weil unterhalb des Reedschalters 21 wie gesagt ein weiterer Permanentmagnet 25 liegt. Die beiden sich anziehenden Magnete 18 und 25 bilden ein Magnetfeld, welches die Kontakte des Reedschalters 21 schliesst. Sobald der Betätigungsmechanismus abläuft und sich die beiden Magnete voneinander entfernen, wird das Magnetfeld abgebaut und der Reedschalter 21 fällt ab. Liegt nachher der andere Permanentmagnet 17 auf dem Eisenkern 16, so stossen sich die beiden gegeneinander gerichteten Nordpole 17 und 25 ab und der Reedschalter 21 schliesst folglich nicht. Das erfindungsgemässe Gerät wird mit einer handelsüblichen alkalinen 9 Volt Batterie 15, welche im Gehäuse 1 untergebracht ist, betrieben. Ist die Batterie zu stark entladen, so vermag das von der Spule 20 aufgebaute Magnetfeld die Anziehungskraft zwischen den Magneten 17 und dem Kern 19 nicht soweit zu neutralisieren, dass die Magnete die Drehplatte 2 nicht mehr gegen die Federkraft zu halten vermögen. Die Drehplatte 2 bleibt deshalb in der Lage nach Figur 2 und die Kontaktlinsen bleiben entsprechend in der Reinigungsflüssigkeit. Eine Neutralisation findet nicht statt. Damit dieses nicht passieren kann oder damit zumindest bei zu schwacher Batterieladung davor gewarnt wird, ist eine Kontoll-Lampe 26 auf der Gehäuseoberseite angebracht, die eine zu niederige Batteriespannung durch Aufleuchten anzeigt. Für die Beschaltung dieser Kontroll-Lampe 26 kann eine integrierte Schaltung des handelsüblichen Typs "Low Batterie Indicator" verwendet werden. Eine solche integrierte Schaltung schaltet bei Abfall der Batteriespannung unter einen gewissen Wert den Strom automatisch auf die Kontroll-Lampe 26. Damit wird dem Benützer angezeigt, dass das Gerät nicht betriebsbereit ist und er also erst die Batterie zu ersetzen hat. Selbstverständlich könnte das erfindungsgemässe Gerät auch mit einer wiederaufladbaren Batterie ausgerüstet sein oder auch mit einem entsprechenden Gleichrichter und Transformer für den Netzbetrieb ausgerüstet sein.

## Patentansprüche

1. Gerät zur Pflege von Kontaktlinsen mittels zwei verschiedenen Flüssigkeiten (3,4), bestehend aus einem Gehäuse (1) mit zwei Behältnissen (8,9) zur Aufnahme der zwei verschiedenen Flüssigkeiten (3,4) und einer darauf beweglich angeordneten Halterung (5) zum abwechslungsweisen und selbsttätigen Eintauchen der von ihr gehaltenen Kontaktlinsen in die beiden Behältnisse (8,9), wobei diese Halterung (5) auf einer Achse bezüglich des Gehäuses (1) schwenkbar gelagert ist; dadurch gekennzeichnet, dass eine Drehplatte (2) vorhanden ist, welche die Halterung (5) trägt, und dass die Drehplatte (2) auf einer Führungsstange (6) gehalten ist, die drehbar und verschiebbar in einer Bohrung (13) im Gehäuse (1) sitzt, welche zwischen den beiden Behältnissen (8,9) angeordnet ist.

2. Gerät nach Anspruch 1, dadurch gekennzeichnet, dass an der Drehplatte (2) parallel zur Führungsstange (6) eine Schubstange (7) angeordnet ist, und dass zwei Bohrungen (14,24) im Gehäuse (1) vorhanden sind, in welche die Schubstange (7) unter zwei verschiedenen Schwenklagen der Drehplatte (2) einschiebbar ist, wobei die Mündungen der beiden Bohrungen (14,24) über eine konzentrisch zur Bohrung (13) verlaufende Führungsnut (12) mit vom Randbereich der Bohrung (14) zur Bohrung (24) hin schraubenlinienförmig abfallendem Nutboden miteinander verbunden sind.

3. Gerät nach Anspruch 2, dadurch gekennzeichnet, dass die Bohrung (14) eine Druckfeder (10) enthält, auf der eine Kugel (11) sitzt, und dass magnetische Mittel vorhanden sind, mittels derer die Drehplatte (2) gegen die Kraft der Druckfeder (10) in ihrer Tieflage, bei welcher die Schubstange (7) die Druckfeder (10) zusammengedrückt hält, festhaltbar ist.

4. Gerät nach Anspruch 3, dadurch gekennzeichnet, dass die magnetischen Mittel Permanentmagnete (17,18), mindestens einen Eisenkern (16) und mindestens einen Elektromagneten (19,20) einschliessen, wobei der Elektromagnet (19,20) von einer elektronisch steuerbaren Stromversorgung gespeist ist.

5. Gerät nach Anspruch 4, dadurch gekennzeichnet, dass zwei Permanentmagnete (17,18) derart in der Drehplatte (2) eingelegt sind, dass der eine mit seinem Nord- und der andere mit seinem Südpol gegen die Unterseite der Drehplatte (2) hin gerichtet ist, ferner dass in der Tieflage der Drehplatte (2), bei welcher die Schubstange (7) die Druckfeder (10) zusammengedrückt hält, die Permanentmagnete (17,18) gegen Eisenkerne (16,19) hin gerichtet sind, die im Gehäuse (1) angeordnet sind, und deren einer (19) von einer Spule (20) umwickelt einen Elektromagneten (19,20) bildet.

6. Gerät nach Anspruch 4 oder 5, dadurch gekennzeichnet, dass die elektronisch steuerbare Stromversorgung einen berührungslosen Reedschalter (21), einen als Zeitelement (23) beschalteten integrierten Schaltkreis (23) und einen Treiber (22) einschliesst, wobei der Reedschalter (21) im Gehäuse (1) unter dem Eisenkern (16) angeordnet ist und unter ihm selbst ein weiterer Permanentemagnet (25) derart angeordnet ist, dass in der Tieflage der Drehplatte (2), bei welcher die Schubstange (7) die Druckfeder (10) zusammengedrückt hält, die Pole der Permanentmagnete (18,25) beidseits des Reedschalters (21) zueinander entgegengesetzt sind.

7. Gerät nach einem der vorhergehenden Ansprüche, dadurch gekennzeichnet, dass die Behältnisse zur Aufnahme der zwei Flüssigkeiten gummielastische Einlegebehältnisse sind, welche in die Behältnisse (8,9) im Gehäuse (1) einlegbar sind und die an ihrem oberen Rand einen Wulst aufweisen, der beim Einlegen auf den Rand der Behältnisse (8,9) zu liegen kommt, derart, dass bei auf die Wulste gedrückter Drehplatte (2) die in den Einlagebehältnissen enthaltenen Flüssigkeiten dichtend abgeschlossen sind.

8. Gerät nach einem der vorhergehenden Ansprüche, dadurch gekennzeichnet, dass am Gehäuse (1) oder an der Drehplatte (2) Schiebeverschlüsse angeordnet sind, mittels denen die Drehplatte (2) in ihrer Tieflage an das Gehäuse (1) pressbar sind.

9. Gerät nach einem der vorhergehenden Ansprüche, dadurch gekennzeichnet, dass am Gerät eine Kontroll-Lampe (26) vorhanden ist, mittels der ein zu schwacher Ladezustand der Gerätbatterie (15) anzeigbar ist.

10. Gerät nach einem der vorhergehenden Ansprüche, dadurch gekennzeichnet, dass im Behälter (8) oder im Einlagebehälter für die Neutralisierungsflüssigkeit ein mit Platinschwamm oder Platinmohr beschichteter Kunststoffkörper angeordnet ist.

## Claims

1. A unit for the care of contact lenses using two different liquids (3,4), consisting of a housing (1) with two receptacles (8,9) for holding the two different liquids (3,4) and a holding device (5) disposed movably thereupon for alternately and automatically dipping the contact lenses held by it into the two receptacles (8,9), this holding device (5) being swivel-mounted on an axis with respect to the housing (1), characterized in that there is a rotating plate (2) which bears the housing device (5), and in that the rotating plate (2) is fixed on a guide rod (6) which rests rotatably and displaceably in a bore (13) in the housing (1), which is disposed between the two receptacles (8,9).

2. The unit of claim 1, characterized in that a connecting rod (7) is disposed on the rotating plate (2) parallel to the guide rod (6), and in that there are two bores (14, 24) in the housing (1), into which the connecting rod (7) can be inserted according to two different swivel positions of the rotating plate (2), the openings of the two bores (14, 24) being connected with each other via a guide groove (12) running concentrically to the bore (13), with the floor of the groove sloping helically from the edge area of bore (14) to bore (24).

3. The unit of claim 2, characterized in that the bore (14) contains a pressure spring (10), on which a ball (11) rests, and in that there are magnetic means by way of which the rotating plate (2) can be held against the force of the pressure spring (10) in its bottom position, in which the connecting rod (7) keeps the pressure spring (10) compressed.

4. The unit of claim 3, characterized in that the magnetic means comprise permanent magnets (17, 18), at least one iron core (16) and at least one electromagnet (19, 20), the electromagnet (19, 20) being fed by an electronically controllable electricity supply.

5. The unit of claim 4, characterized in that two permanent magnets (17, 18) are inserted in the rotating plate (2) in such a way that the north pole of one and the south pole of the other is directed towards the underside of the rotating plate (2), furthermore in that when the rotating plate (2) is in the bottom position, in which the connecting rod (7) keeps the pressure spring (10) compressed, the permanent magnets (17, 18) are directed towards the iron cores (16, 19) which are disposed in the housing (1), and of which one (19), enclosed by a coil (20), forms an electromagnet (19, 20).

6. The unit of claim 4 or 5, characterized in that the electronically controllable electricity supply comprises a non-contacting reed switch (21), an integrated switching circuit (23) wired up as a timing element (23) and a driver (22), the reed switch (21) being disposed in the housing (1) under the iron core (16) and a further permanent magnet (25) being disposed under it itself such that when the rotating plate (2) is in the bottom position, in which the connecting rod (7) keeps the pressure spring (10) compressed, the poles of the permanent magnets (18, 25) on both sides of the reed switch (21) are opposed to each other.

7. The unit of one of the preceding claims, characterized in that the receptacles for holding the two liquids are insertable rubber-elastic receptacles which can be inserted into the receptacles (8,9) in the housing (1) and which have a lip at their top edge which, on insertion, comes to rest on the edge of receptacles (8,9) such that when the rotating plate (2) is pressed down on the lip, the liquids contained in the insertable receptacles are sealed off.

8. The unit of one of the preceding claims, characterized in that sliding locks are disposed on the housing (1) or on the rotating plate (2), by means of which the rotating plate (2) can be pressed against the housing (1) when in its bottom position.

9. The unit of one of the preceding claims, characterized in that there is a control lamp (26) on the unit, by means of which an insufficient charge on the unit's battery (15) can be indicated.

10. The unit of one of the preceding claims, characterized in that a plastic element coated with platinum sponge or platinum black is disposed in the receptacle (8) or in the insertable receptacle for the neutralizing liquid.

## Revendications

1. Appareil pour l'entretien de lentilles de contact au moyen de deux liquides différents (3, 4), constitué par un boîtier (1), comportant deux récipients (8, 9), destinés à recevoir les deux liquides (3, 4), et une fixation (5) disposée mobile par-dessus, destinée à plonger alternativement et indépendamment dans les deux récipients (8, 9) les lentilles de contact tenue par elle, cette fixation (5) étant logée pivotante sur un axe par rapport au boîtier (1), caractérisé en ce qu'il existe une plaque tournante (2), portant la fixation (5) et que la plaque tournante (2) est maintenue sur une tige de guidage (6), logée tournante et déplaçable dans un alésage (13) du boîtier (1), cet alésage étant disposé entre les deux récipients (8, 9).

2. Appareil suivant la revendication 1, caractérisé en ce que sur la plaque tournante (2) et parallèlement à la tige de guidage (6) une tige de poussée (7) est disposée et qu'il existe dans le boîtier (1) deux alésages (14, 24), dans lesquels la tige de poussée (7) peut être insérée dans deux positions de pivotement différentes de la plaque tournante (2), cependant que les orifices des deux alésages (14, 24) sont reliés entre eux par une rainure de guidage (12), de tracé concentrique par rapport à l'alésage (13) et comportant un fond de rainure descendant hélicoïdalement de la bordure de l'alésage (14) vers l'alésage (24).

3. Appareil suivant la revendication 2, caractérisé en ce que l'alésage (14) contient un ressort de compression (10), sur lequel est logée une bille (11) et qu'il existe des moyens magnétiques, grâce auxquelles la plaque tournante (2) peut être fixée contre l'action du ressort (10) dans sa position basse, dans laquelle la tige de poussée (7) maintient comprimé le ressort de compression (10).

4. Appareil suivant la revendication 3, caractérisé en ce que les moyens magnétiques comprennent des aimants permanents (17, 18), au moins un noyau de fer (16) et au moins un électro-aimant (19, 20), ce dernier étant alimenté par une alimentation commandable électroniquement.

5. Appareil suivant la revendication 4, caractérisé en ce que deux aimants permanents (17, 18) sont insérés dans la plaque tournante (2), de sorte que l'un est orienté avec son pôle nord et l'autre avec son pôle sud vers le dessous de la plaque tournante (2), que par ailleurs en position basse de la plaque tournante (2), dans laquelle la tige de poussée (7) maintient comprimé le ressort de compression (10), les aimants permanents (17, 18) sont orientés vers des noyaux de fer (16, 19), disposés dans le boîtier (1) et dont l'un (19) est enveloppé par une bobine (20), formant ainsi un électro-aimant (19, 20).

6. Appareil suivant la revendication 4 ou 5, caractérisé en ce que l'alimentation commandable électroniquement comprend un commutateur reed sans contact (21), un circuit intégré (23) connecté en élément de temporisation et un étage pilote (22), cependant que le commutateur reed (21) est disposé dans le boîtier (1), sous le noyau de fer (16), tandis que sous lui-même est disposé un autre aimant permanent (25), de telle sorte qu'en position basse de la plaque tournante (2), dans laquelle la tige de poussée (7) maintient compressé le ressort de compression (10), les pôles des aimants permanents (18, 25) sont opposés entre eux, des deux côtés de commutateur reed (21).

7. Appareil suivant une des revendications précédentes, caractérisé en ce que les récipients destinés à recevoir les deux liquides sont des récipients insérés, ayant l'élasticité du caoutchouc, qui peuvent être insérés dans les récipients (8, 9) se trouvant dans le boîtier (1) et comportent un bourrelet sur leur bord supérieur, ce bourrelet venant, au moment de l'insertion, s'appliquer sur le bord des récipients (8, 9), de sorte que, quand la plaque tournante (2) est appuyée sur les bourrelets, les liquides contenus dans les récipients insérés sont enfermés hermétiquement.

8. Appareil suivant une des revendications précédentes, caractérisé en ce que sur le boîtier (1) ou sur la plaque tournante (2) il est disposé des dispositifs de fermeture coulissants, au moyen desquels la plaque tournante (2) peut être appuyée dans sa position basse contre le boîtier (1).

9. Appareil suivant une des revendications précédentes, caractérisé en ce qu'il est disposé sur l'appareil une lampe témoin (26), qui permet de signaler une charge trop faible de la batterie (15) de l'appareil.

10. Appareil suivant une des revendications précédentes, caractérisé en ce que dans le récipient (8) ou dans le récipient à insérer recevant le liquide de neutralisation il est disposé un corps en matière plastique, enduit de mousse de platine ou d'éponge de platine.
